# EUROPÄISCHE PATENTANMELDUNG

(11) **EP 3 141 329 A1**
(43) Veröffentlichungstag der Anmeldung: **15.03.2017**
(21) Anmeldenummer: 15002622.7
(22) Anmeldetag: 08.09.2015
(51) Int. Cl.: B23K 9/32, A41D 13/00, A61F 9/06, B23K 37/00, F16P 1/06

(54) **VERFAHREN UND VORRICHTUNG ZUM FÜGEN ODER TRENNEN MIT EINER PERSÖNLICHEN SCHUTZAUSRÜSTUNG**

(71) Anmelder: Linde Aktiengesellschaft, 80331 München (DE)
(72) Erfinder: Siewert, Erwan, 85283 Niederlauterbach (DE); Trautmann, Andreas, 80805 München (DE); Büchele, Wolfgang, 80331 München (DE); Blades, Thomas, 80331 München (DE)
(74) Vertreter: Gellner, Bernd

(57) **Zusammenfassung**

Die Erfindung betrifft ein Verfahren und eine Vorrichtung zum Fügen oder Trennen, wobei eine das Füge- oder Trennverfahren durchführende Person mit einer persönlichen Schutzausrüstung ausgestattet ist. Zumindest ein Teil der das Füge- oder Trennverfahren charakterisierenden Daten wird erfasst einem zentralen Prozessor, der in die persönliche Schutzausrüstung integriert ist, als Eingangsdaten zur Verfügung gestellt werden. Die erzeugten Ausgangsdaten werden dazu eingesetzt, um das Verfahren zu steuern, wobei die Steuerung durch die durchführende Person ergänzt wird.

## Beschreibung

Die Erfindung betrifft ein Verfahren und eine Vorrichtung zum Fügen oder Trennen, wobei eine das Füge- oder Trennverfahren durchführende Person mit einer persönlichen Schutzausrüstung ausgestattet ist und wobei zumindest ein Teil das Füge- oder Trennverfahren charakterisierender Daten und/oder zumindest ein Teil der bei der Durchführung des Füge- oder Trennverfahrens entstehenden Emissionen erfasst werden und die erfassten Informationen einem zentralen Prozessor als Eingangsdaten zur Verfügung gestellt werden, und wobei der zentrale Prozessor die Eingangsdaten verarbeitet und Ausgangsdaten erzeugt.

### Stand der Technik

Innerhalb der Fügeverfahren dominieren Verfahren, die einen Lichtbogen als Energiequelle verwenden. Technisch sehr ähnliche Verfahren wie das thermische Beschichten, das Löten oder das Generieren von Strukturen werden häufig als "verwandte Verfahren" bezeichnet. Wird im Folgenden von einem Füge- oder Trennverfahren gesprochen, sollen daher neben Schweißprozessen auch die verwandten Verfahren umfasst sein. Als Energiequelle kann neben einem Lichtbogen auch ein Laser, eine Flamme oder ein Elektronenstrahl zum Einsatz kommen ohne den Rahmen der Erfindung zu verlassen. Ebenfalls umfasst sollen Trennverfahren sein, wie z.B. das Laserschneiden, das Plasmaschneiden und das Flammschneiden.

Dem Fachmann sind aus dem Stand der Technik unterschiedliche Schweißverfahren bekannt, die sich jeweils für bestimmte schweißtechnische Aufgaben in besonderer Weise eignen. Eine Übersicht geben beispielsweise Dilthey, U.: Schweißtechnische Fertigungsverfahren 1: Schweiß- und Schneidtechnologien. 3. Aufl. Heidelberg: Springer, 2006 oder Davies, A.C.: The Science and Practice of Welding. 10. Aufl. Cambridge: Cambridge University Press, 1993. Die am häufigsten eingesetzten Verfahren sind das WIG-, das MSG- und das Plasmaschweißen. Zudem werden Laser zum Schweißen sowie in Kombination mit den oben genannten Lichtbogenprozessen eingesetzt

Beim Wolfram-Inertgasschweißen (WIG-Schweißen, engl. Tungsten Gas Welding, TIG) brennt ein Lichtbogen zwischen einer nicht abschmelzenden Wolframelektrode und dem zu bearbeitenden Werkstück. Das Werkstück wird hierdurch aufgeschmolzen. Zum Schutz der Wolframelektrode und des sich ausbildenden Schmelzbads vor Oxidation wird ein geeignetes Schutzgas eingesetzt, das die Wolframelektrode und das Schmelzbad abdeckt. Beim WIG-Schweißen wird typischerweise in inerter, seltener in reduzierender Atmosphäre gearbeitet.

Durch das WIG-Verfahren lässt sich typischerweise eine sehr hohe Schweißqualität erreichen. Jedoch sind diese nicht beliebig automatisierbar und ermöglichen insbesondere im Vergleich zu den nachfolgend erläuterten Verfahren nur eine vergleichsweise geringe Produktivität aufgrund der geringeren Schweißgeschwindigkeit und Abschmelzleistung.

Beim Metall-Schutzgasschweißen (MSG, engl. Gas Metal Arc Welding, GMAW) wird dem Schweißbrenner eine Drahtelektrode, die damit auch gleichzeitig einen Schweißzusatzwerkstoff bildet, kontinuierlich zugeführt und in einem Lichtbogen abgeschmolzen. Es wird ebenfalls ein Schutzgas verwendet. Je nach Art des Schutzgases unterscheidet der Fachmann zwischen Metall-Inertgasschweißen (MIG, engl. Metal Inert Gas Welding) und Metall-Aktivgasschweißen (MAG, engl. Metal Active Gas Welding). Die grundlegenden Verfahrensprinzipien gleichen einander. Typischerweise werden einem MSG-Brenner der Schweißstrom, die Drahtelektrode, das Schutzgas und ggf. erforderliches Kühlwasser durch ein Schlauchpaket zugeführt.

MSG-Verfahren erlauben eine hohe Schweißgeschwindigkeit und damit eine überlegene Produktivität im Vergleich zu WIG-Verfahren. Die Automatisierbarkeit von MSG-Verfahren ist ausgesprochen hoch. Nachteilig an der Verwendung des direkt im Lichtbogen erhitzten, aufgeschmolzenen und verdampften Zusatzwerkstoffs ist die wesentlich höhere Emission von Partikeln im Vergleich zu WIG-Verfahren. Die mit MSG-Verfahren erzielbare Schweißqualität wird im Vergleich zu WIG-Verfahren häufig als geringer angesehen. Sonderverfahren nutzen mehrere Elektroden. Hierbei werden am häufigsten zwei Drahtelektroden eingesetzt (Tandem-Schweißen).

Im Gegensatz zu den genannten Verfahren, bei denen der Lichtbogen frei brennt, wird dieser bei den ebenfalls bekannten Plasmaschweißverfahren eingeschnürt, wofür i.d.R. wassergekühlte Kupferdüsen zum Einsatz kommen. Dies führt zu einer Verringerung des Lichtbogenquerschnitts. Beim Plasmaschweißen wird mit einer nicht abschmelzenden Elektrode gearbeitet. Die zur Bildung eines Plasmas erforderlichen Ladungsträger werden durch die Ionisierung eines inerten Plasmagases (Argon oder Gemischen aus Argon, Helium und/oder Wasserstoff) geliefert.

Beim Plasmaschweißen mit nicht abschmelzender Elektrode kann ein Lichtbogen innerhalb des Schweißbrenners und/oder zwischen dem Schweißbrenner und dem Werkstück ausgebildet werden (sogenannter "nicht übertragener" bzw. "übertragener" Lichtbogen). Man unterscheidet zwischen Plasmastrahlschweißen (WPS, nicht übertragener Lichtbogen), Plasmalichtbogenschweißen (WPL, übertragener Lichtbogen) und dem kombinierten Plasmastrahl-Plasmalichtbogenschweißen (WPSL, sowohl nicht übertragener als auch übertragener Lichtbogen).

Beim sogenannten Plasma-MIG-Schweißen wird eine abschmelzende Elektrode eingesetzt und ein Plasmalichtbogen zwischen einer Plasmagasdüse und dem bearbeiteten Werkstück gezündet. Der Plasmalichtbogen wird mittels einer sogenannten Fokussiergasdüse und unter Verwendung eines entsprechenden Fokussiergases eingeschnürt. Die Plasmagasdüse, die Fokussiergasdüse und die ebenfalls vorhandene Schutzgasdüse sind koaxial angeordnet. Die wie bei herkömmlichen MSG-Verfahren eingesetzte abschmelzende Drahtelektrode wird zentrisch zugeführt. Sowohl die Plasmagasdüse als auch die Drahtelektrode liegen auf positivem Potential und werden üblicherweise von getrennten Stromquellen gespeist. Weitere Beispiele für hybride Verfahren kombinieren ein Laser- mit einem Lichtbogenverfahren.

Die oben genannten Verfahren geben Informationen in Form von Emissionen, wie z.B. Licht, Schall, elektromagnetische Strahlung sowie partikel- und gasförmige Stoffe, ab. Diese Emissionen unterscheiden sich erheblich hinsichtlich ihrer Art und Menge in Abhängigkeit von dem eingesetzten Verfahren. Weiterhin unterscheiden sich diese Prozesse durch ihre Art des Materialtransfers und daher durch ihr dynamisches Verhalten. So schmilzt z.B. die Elektrode beim WIG-Schweißen nicht, sodass ein sehr konstanter Lichtbogen ohne nennenswerte Helligkeitsunterschiede entsteht, wohingegen die Elektrode beim MSG-Schweißen zugleich Zusatzmaterial ist und abschmilzt, sodass es z.B. durch den Materialtransfer oder durch Kurzschlüssen zu einem hochdynamischen Lichtbogenverhalten mit sehr großen Helligkeitsunterschieden kommt. Dadurch fallen die Möglichkeiten zur Beobachtung und zur Regelung der verschiedenen Schweißprozesse sehr unterschiedlich aus. Trotz dieser deutlichen Unterschiede in den Schweißverfahren werden in der Regel immer identische Schutzausrüstungen eingesetzt. Für das manuelle Lichtbogenschweißen werden Schweißerschutzhelme oder Schweißerschutzschilde verwendet, welche den Schweißer vor Hitze, Schweißspritzern und Strahlung schützen. Neuste Helme messen die Intensität der Strahlung und passen anhand dieser Informationen den Transmissionsgrad des Sichtfensters automatisch an (z.B. Fa. Optrel). Immer häufiger, aber zumeist nur bei sehr speziellen Anwendungen, werden fremdbelüftete Helme eingesetzt, welche für eine Reduzierung der gas- und partikelförmigen Emissionen im Atembereich des Anwenders sorgen, den Bediener jedoch auch in seiner Tätigkeit einschränkt.

Das Einstellen und die Kontrolle des Schweißprozesses erfolgt beim manuellen Schweißen überwiegend visuell und akustisch basierend auf den Erfahrungen des Schweißers. Der Informationsgehalt wird durch die Leistungsfähigkeit der menschlichen Sinnesorgane limitiert. Für gasförmige wie partikelförmige Emissionen besitzt der Mensch teilweise gar keine geeigneten Sinne, bzw. reagiert hier der Körper erst, wenn bereits eine Schädigung eingetreten ist (Reizung der Atemwege / Schleimhäute, Fieber, etc.). Auch bei den sehr konzentrierten Strahlungsemissionen eines Lasers, kann der Körper in der Regel nicht mehr rechtzeitig reagieren, bevor eine Schädigung einsetzt.

Eine zunehmende Automatisierung sowie Digitalisierung und insbesondere der vermehrte Einsatz von Robotersystemen, führen zu einer zunehmenden Anzahl an Informationen, welche vom Bediener erfasst und verarbeitet werden müssen. Insbesondere ist die Differenzierung zwischen wichtigen und unwichtigen Informationen bei der auftretenden Datenflut schwierig. Dem Bediener ist es nur schwer möglich, sich neben dem Schweißvorgang auch noch auf die Bewegungsvorgänge oder auf andere Peripheriegeräte und Sensoren zu konzentrieren, sodass es zunehmend zu einer Überforderung des Bedienpersonals kommt.

Momentan werden die Positioniersysteme daher vor dem Schweißen, ohne das Einschalten des Lichtbogens programmiert und folgen beim Schweißvorgang genau den vorher eingegebenen Werten, ohne auf Prozessunregelmäßigkeiten beim Schweißen reagieren zu können. Die zunehmende Automatisierung und die steigende Anzahl an Bedien- und Peripheriesystemen, welche sich zudem von Arbeitsplatz zu Arbeitsplatz und von Fügeaufgabe zu Fügeaufgabe verändern, bedingt einen steigenden Aufwand für den Aufbau, die Koppelung, die Programmierung sowie für die Einarbeitung in die entsprechenden Systeme. Andererseits nimmt die Anzahl an qualifiziertem Bedienpersonal stetig ab, sodass der Bedarf an Systemen, welche einen unerfahrenen Schweißer unterstützen, sodass dieser eine Schweißverbindungen in der geforderten Qualität produzieren kann, zunimmt.

Beim automatisierten Schweißen werden teilweise Kamerasysteme eingesetzt, welche es erlauben, den Schweißvorgang (von einer von der Schweißstelle entfernten Position) zu beobachten und ggf. zu dokumentieren. Diese externen Sensoren, welche in der Schweißtechnik eingesetzt werden, sind in ihrer Bandbreite, Frequenz und Dynamik stark limitiert, da diese mit dem Ziel entwickelt wurden, die Eigenschaften unserer Sinnesorgane möglichst gut abzubilden. Somit können diese Sensoren in der Regel auch nicht mehr bzw. nicht viel mehr Informationen liefern, als der Mensch erfassen würde. In einigen Fällen sind diese Sensoren den menschlichen Sinnesorganen sogar unterlegen (z.B. Dynamikumfang der Strahlungsintensität bei gewöhnlichen CCD und CMOS Kameras beträgt häufig nur 1:1000). Zudem werden beim Schweißen fast ausschließlich bildgebende Sensoren eingesetzt und somit nur ein sehr kleiner Teil der Emissionsinformationen genutzt.

In heutigen Schweißprozessen sitzt die Steuer- und Regeleinheit - und damit die Intelligenz - für den Schweißprozess in der Schweißstromquelle. Diese Stromquellen bieten die Möglichkeit Daten sehr schnell zu erfassen und zu verarbeiten. Hierbei besteht jedoch der Nachteil, dass sich die Stromquelle in einer zu großen Distanz vom Bedienpersonal befindet, sodass es nicht möglich ist, wichtige Informationen auf dem Display der Schweißstromquelle während des Fügevorgangs zu erfassen bzw. Änderungen am Schweißprozess während des Schweißvorgangs vorzunehmen. Bedienpanel oder Bedienelemente am Brenner ermöglichen nur geringfügige Verbesserungen. Weiterhin befinden sich die Sensoren, welche für die Überwachung und die Steuerung und/oder Regelung des Prozesses herangezogen werden direkt in der Stromquelle, sodass die Signale des Lichtbogens, welche für den Prozess ausschlaggebend sind, über die Distanz gestört, verzögert und abgeschwächt werden. Weiterhin werden nur die beiden elektrischen Signale Strom und Spannung erfasst, welche jedoch nur sehr limitierte Informationen liefern können.

Bisher ist es daher noch nicht (oder nur sehr eingeschränkt) möglich, anhand dieser Informationen auf die Qualität des Schweißergebnisses Rückschlüsse zu ziehen. So ist z.B. das Auftreten von Poren oder Bindefehlern nicht anhand dieser Größen zu detektieren.

Ferner werden auch nur die Schweißstromstärke oder die Klemmenspannung sowie in speziellen Fällen der Drahtvorschub geregelt. Wird der Prozess anhand dieser Daten geregelt, erfolgt eine Zuordnung nach dem Ursache-Wirkungsprinzip, wobei bei allen bekannten Techniken ein monokausaler Zusammenhang zugrunde gelegt wird. Der Stand der Technik basiert daher ausnahmslos auf der Lehre eineindeutiger Korrelationen (Bijektionen). Die Kausalität (z.B. Lichtbogenspannung) wird einer Auswirkung (z.B. Schweißnahtfehler) zugeordnet. Mehrere unterschiedliche Informationen werden nicht für die Beurteilung einer Auswirkung herangezogen.

Das Einhalten von Grenzwerten am Arbeitsplatz wird in zumeist sehr aufwändigen Messungen überprüft. Problematisch ist hierbei, dass sich die Bedingungen am Arbeitsplatz ständig ändern. Die Änderungen werden unter anderem durch eine wechselnde Arbeitsumgebung, ein anderes zu schweißendes Bauteil, einen anderen Schweißprozess, unterschiedliche Schweißparameter oder auch eine unterschiedliche Körperposition hervorgerufen. Diese Messungen müssen zudem zumeist aufwändig im Labor ausgewertet werden und stehen erst nach einigen Stunden bis Tagen zur Verfügung.

Das Aufzeigen von Belastungen im Atembereich des Schweißers unter Produktionsbedingungen und in "real-time" ist derzeit nicht möglich. Die Grenzwerte für Gefahrstoffe beim Schweißen wurden in der Vergangenheit drastisch reduziert. Es ist abzusehen, dass sich dieser Trend fortsetzt, sodass die Schweißtechnik und der Gesundheitsschutz vor neuen Herausforderungen stehen, dieses Problem zu lösen.

Momentan wird ein enormer Aufwand betrieben, alle möglichen Maßnahmen zu treffen, um die Immission für den Menschen möglichst gering zu halten. Zusätzlich werden die Auswirkungen auf den Schweißer in kontinuierlichen Untersuchungen des Urins und / oder des Blutes überwacht und dokumentiert. Grenzwerte beim Schweißen und Maßnahmen zur Bestimmung der Arbeitsplatzkonzentration finden sich in den Informationen der BG Holz und Metall

Aufgabe vorliegender Erfindung ist es daher, ein Verfahren und eine Vorrichtung zum Fügen oder Trennen aufzuzeigen, welche die oben genannten Nachteile möglichst vermeiden.

### Offenbarung der Erfindung

Die gestellte Aufgabe wird durch ein Verfahren zum Fügen oder Trennen nach Anspruch 1 und eine Vorrichtung mit den Merkmalen des Anspruchs 10 gelöst.

Das erfindungsgemäße Verfahren zum Fügen oder Trennen wird einerseits von einer mit der Durchführung des Verfahrens betrauten Person gesteuert, andererseits erfolgt die Steuerung des Verfahrens auch mittels eines zentralen Prozessors. Zumindest ein Teil das Füge- oder Trennverfahren charakterisierender Daten und/oder zumindest ein Teil der bei der Durchführung des Füge- oder Trennverfahrens entstehenden Emissionen wird, beispielsweise mittels eines oder mehrerer Sensoren, erfasst und die erfassten Informationen werden dem zentralen Prozessor als Eingangsdaten zur Verfügung gestellt. Der zentrale Prozessor verarbeitet die Eingangsdaten und erzeugt Ausgangsdaten, welche zumindest zum Teil dazu eingesetzt werden, um das Verfahren zu steuern. Der zentrale Prozessor ist dabei in die persönliche Schutzausrüstung der das Verfahren durchführenden Person integriert.

Unter einer persönlichen Schutzausrüstung ist im Rahmen der vorliegenden Erfindung eine am Körper getragenen Schutzausrüstung für eine Person zu verstehen, wie z.B. ein Schutzhelm (im folgenden auch kurz als Helm bezeichnet), ein Schutzschild, eine Schutzbrille, Schutzhandschuhe oder ein Schutzanzug. Es sollen also die Augen, die Atemwege, die Ohren, das Gesicht und/oder die Haut der das Verfahren durchführenden Person mit einem Voll- oder Teilschutz vor diesen schädigenden Einflüssen geschützt werden.

Mit den Begriffen "steuern" und "Steuerung" sollen hier jeweils auch die Begriffe "regeln" und "Regelung" mit umfasst sein. Aus Gründen der besseren Lesbarkeit des Textes wird meist lediglich der Begriff "steuern" verwendet.

Die persönliche Schutzausrüstung mit dem zentralen Prozessor, der auch als zentrale Recheneinheit, Rechner oder Mikroprozessor bezeichnet bzw. ausgeführt sein kann, nehmen durch die Erfindung erstmals eine zentrale Funktion für den gesamten Fügevorgang ein, die man auch als Schlüsselfunktion bezeichnen kann, welche bisher mit keinem anderen Gerät oder durch keine andere Technik gelöst werden konnte.

Die Funktionen der Schutzausrüstung mit dem integrierten Prozessor übersteigen die reinen Schutzfunktionen, wie beispielsweise eine automatische Verringerung des Transmissionsgrads des Sichtfensters bei zu hoher Strahlungsintensität. Erfindungsgemäß dient der in die Schutzausrüstung integrierte Prozessor nicht nur zur Steuerung der Schutzfunktionen, sondern auch zur Steuerung des Füge- oder Trennverfahrens. Die von dem zentralen Prozessor erzeugten Ausgangsdaten werden zumindest teilweise genutzt, um direkt in die Steuerung des Füge- oder Trennverfahrens einzugreifen. Bisher werden die Ausgangsdaten der durchführenden Person nur angezeigt. Erfindungsgemäß werden die Ausgangsdaten jedoch zumindest teilweise zur Änderung, Anpassung oder Optimierung von Prozessparametern des Verfahrens herangezogen. Der zentrale Prozessor wirkt unmittelbar auf die Steuerung des Verfahrens ein.

Das Füge- oder Trennverfahren wird zum einen von der durchführenden Person gesteuert, zum anderen von dem zentralen Prozessor, wobei letzterer direkt auf die Steuerung einwirken kann oder der durchführenden Person beispielsweise über eine entsprechende Anzeige Vorschläge für eine Anpassung der Steuerung machen kann oder der durchführenden Person, beispielsweise über eine entsprechende Anzeige, lediglich die Ausgangsdaten mitteilt und eventuelle Aktionen der durchführenden Person überlässt.

Neben der Erfassung von das Füge- oder Trennverfahren charakterisierender Daten und/oder Emissionen können auch weitere externe Geräte in den Kommunikationskreis des zentralen Prozessors eingebunden werden. Die externen Geräte oder Peripheriegeräte senden dann ihre Daten an den zentralen Prozessor. Die an den Prozessor geschickten Daten werden aufbereitet, gegebenenfalls dargestellt und gegebenenfalls direkt in Eingriffe in die Steuerung umgesetzt. Letzteres kann zum Beispiel dadurch erfolgen, dass der Prozessor Befehle an die Peripheriegeräte schickt.

Der Datenverarbeitung wird mit Hilfe des zentralen Prozessors bevorzugt in Echtzeit durchgeführt.

Als Beispiele für die zwischen den externen Geräten oder Peripheriegeräten und dem zentralen Prozessor ausgetauschten Daten oder Prozessparameter können angegeben werden:
Kinematik der Positionier- und Haltesysteme für das zu fügende Werkstück und/oder das Fügewerkzeug (wie z.B. Schweißbrenner),
Strom und Spannung der Energieversorgung des Verfahrens (wie z.B. Schweißstrom, Schweißspannung),
Vorschub oder Mengenverbrauch von Schweißzusatzwerkstoffen (wie z.B. Draht, Pulver, Fülldraht),
Temperatur, Druck, Mengendurchfluss und/oder Zusammensetzung eines Kühlmediums (wie z.B. Wasser, Gas, Luft, andere Flüssigkeiten, Gasgemische), optische Eigenschaften des Lichtbogens oder einer zum Einsatz kommenden Laserstrahlung,
Aussehen oder Temperatur der Füge- oder Trennnaht oder des Schmelzbades am zu fügenden oder zu trennenden Werkstück,
Zusammensetzung der Atemluft im Gesichtsbereich der das Verfahren durchführenden Person,
Zusammensetzung der Raumluft in dem Raum, in dem das Verfahren durchgeführt wird,
Infrarotstrahlung, die das Verfahren emittiert.

Diese Aufzählung erhebt keinen Anspruch auf Vollständigkeit, vielmehr sind eine Reihe weiterer Prozessparameter denkbar, deren Erfassung mit einem oder mehreren Sensoren und Nutzung der erfassten Messdaten - mit Hilfe des zentralen Prozessors - zur Verbesserung der Prozesssteuerung eines erfindungsgemäßen Verfahrens besonders vorteilhaft ist.

Als geeignete Sensoren dienen hierzu die bekannten Sensoren für die genannten Größen sowie Digitalkameras oder hochauflösende Digitalkameras.

Im Arbeitsspeicher des zentralen Prozessors können ein Algorithmus zur Verarbeitung der Eingangsdaten sowie weitere für den Algorithmus benötigte Daten, wie z.B. Tabellenwerte, hinterlegt sein. Diese Informationen können aber auch aus einem weiteren mit dem Prozessor vernetzten Datenspeicher oder einer anderen Quelle für Daten entnommen werden.

Durch die Erfindung wird die Informationsaufnahme, die Intelligenz (Verarbeitung) und die Steuerung und teilweise die Visualisierung an einem Ort, nämlich der Schutzausrüstung, und konkret beispielsweise im Helm, gebündelt.

Die Sensoren und die nachgeschaltete Aufbereitung der Messsignale, d.h. die Verarbeitung der Eingangsdaten und Erzeugung von Ausgangsdaten, können neben der Prozessoptimierung und der Steuerung und/oder Regelung des Schweißprozesses auch zu einer Qualitätskontrolle und/oder Qualitätsdokumentation des Prozesses genutzt werden.

Die Sensoren und die nachgeschaltete Aufbereitung und Korrelation der Messsignale kann neben der Qualitätskontrolle und der Regelung des Schweißprozesses auch zu einer Optimierung des Prozesses genutzt werden. Unter Aufbereitung der Messdaten bzw. Verarbeitung der Eingangsdaten wird beispielsweise die Synchronisierung, d.h. die zeitliche Zuordnung der Messsignale verstanden sowie die Informationsverdichtung, z.B. durch Glättungen, durch das Einbeziehen von differentiellen Größen, dem Zusammenführen von verschiedenen Signalen (Emissionsarten) sowie auch durch Entfaltungstechniken wie z.B. Regressionsanalysen. Zur Deutung der Eingangs- und/oder Ausgangsdaten können Methoden der explorativen Datenanalyse und der inversen Modellierung Einsatz finden. Durch z.B. numerische Ableitungen, Phasenraumdarstellungen oder Vergleiche mit Hüllkurven wird es möglich Ereignisse zu detektieren sowie z.B. das Hystereseverhalten des Systems und damit die Stabilität und Qualität zu beurteilen.

Die beschrieben Operationen werden automatisch anhand von Algorithmen durchgeführt. Ggf. muss der Schweißer bzw. allgemein die durchführende Person hierfür vor der Schweißung bzw. allgemein dem Füge- oder Trennverfahren grundlegende Angaben machen, wie z.B. den Schweißprozess oder das zu fügende Material.

Bevorzugt ist mindestens ein Sensor in die persönliche Schutzausrüstung integriert. Dadurch wird vorteilhaft sichergestellt, dass die Bedingungen im Nahbereich der das Verfahren durchführenden Person präzise und unverfälscht erfasst werden können. Beispielsweise wird die Konzentration eines die Gesundheit des Menschen beeinträchtigenden Gases im Nahbereich der Person gemessen. Durch die Raumluft verdünnt würde eine weiter entfernte Messung an anderer Stelle im Raum womöglich einen geringeren Wert ermitteln. Eine real vorhandene Gefährdung der Person könnte so eventuell nicht erkannt werden.

Ein oder mehrere Sensoren zur Erfassung der Emissionen können auch an einem Brenner zur Durchführung des Füge- oder Trennverfahrens vorgesehen sein. Beispielsweise kann ein solcher Sensor direkt am Schweißbrenner angebracht sein.

Erfindungsgemäß kommunizieren bzw. tauschen die Sensoren auch untereinander Daten aus und können auch eigene Prozessoren besitzen (wie z.B. bei intelligenten Kameras), um eine Signalverarbeitung vorzunehmen.

Wie vorstehend bereits erwähnt, muss die Kommunikation zwischen dem Prozessor und etwaigen Sensoren oder externen Geräten nicht kabellos erfolgen. Es können auch Kabel zur Datenübertragung verwendet werden, bzw. das Stromversorgungsnetz auch zur Kommunikation bzw. zum Datentransfer genutzt werden.

Besonders bevorzugt werden zwei oder mehr als zwei Sensoren eingesetzt, die jeweils zur meßtechnischen Erfassung einer unterschiedlichen Emissionsart des Füge- oder Trennverfahrens geeignet ausgebildet sind. Die so gewonnenen Messwerte werden dem zentralen Prozessor als Eingangsdaten zur Verfügung gestellt, mit Hilfe eines im Arbeitsspeicher hinterlegten Algorithmus verarbeitet und die durch diese Datenverarbeitung erzeugten Ausgangsdaten zur Steuerung des Verfahrens eingesetzt.

Der oder die Sensor(en) können dabei beispielsweise die Atemluft im Bereich unmittelbar vor dem Gesicht der den Füge- oder Trennprozess durchführenden Person überwachen. Die so aufgenommenen Daten werden zur Steuerung einzelner Prozessparameter verwendet, um so bei einer bestimmte Vorgaben unterschreitenden Qualität der gemessenen Atemluft eine Optimierung des Füge- oder Trennverfahrens zu erzielen, bei dem die Entstehung von Stoffen reduziert wird, die die Qualität der Atemluft verringern.

Beispielsweise wird gemäß der vorliegenden Erfindung eine Größe gemessen oder aufgenommen, von der aus man auf die Qualität der Schweißverbindung Rückschlüsse ziehen kann. Die Rückschlüsse werden mit Hilfe des zentralen Prozessors und gegebenenfalls mit Hilfe des hinterlegten Algorithmus gezogen. Das Ergebnis dient der Prozesssteuerung: Erfüllt die ermittelte Qualität die Anforderungen der gestellten Füge-oder Trennaufgabe, wird nicht in den Prozess eingegriffen; Ist die ermittelte Qualität zu gering, wird über die Prozesssteuerung in den Füge- oder Trennprozess eingegriffen und mindestens ein Prozessparameter verändert, um die Qualität auf das geforderte Niveau zu bringen.

Der zentrale Prozessor nimmt dabei die Schlüsselstelle mit einer Vielzahl von Aufgaben ein: als Zentrale für Information, Auswertung von Messdaten, die von den Sensoren bereitgestellt werden, Berechnung von Prozessparametern, die der Prozesssteuerung - und damit einer Steuereinheit - zur Verfügung gestellt werden, wobei über Steuerung und Datenerfassung Regelkreisläufe entstehen können, die eine beonders vorteilhafte Prozessführung ermöglichen.

Der zentrale Prozessor kann auch der Qualitätsüberwachung dienen. Hierfür ist er mit besonderem Vorteil noch mit einem externen Datenspeicher vernetzt.

Beispiele von Sensoren sind neben den bekannten bildgebenden Sensoren auch Füllstands- und Drucksensoren einer Gasflasche, welche zudem Daten wie das TüV Datum oder die Gaszusammensetzung oder auch die aktuelle Gasabnahmemenge liefern. Auch Informationen der Positioniersysteme können an den zentralen Prozessor gesendet werden, um Positionen, Geschwindigkeiten oder bevorstehende Bewegungen anzuzeigen. Die Schweißmaschine kann Daten wie den Schweißstrom, die Schweißspannung, den Drahtvorschub oder die Gas- und Kühlwasserdurchflüsse liefern. Denkbar sind zudem Temperatursensoren im Kühlwasserkreislauf oder Temperatursensoren am Brenner, sodass auch die Brennerbelastung bestimmt werden kann.

Zusätzlich können sich auch Sensoren am Helm, an einer Brille, an einem Schild bzw. im Raum, am Körper des Schweißers oder am Schweißbrenner oder an der Positioniervorrichtung (Schweißportal, Roboter, Drehtisch, etc.) befinden, um die elektromagnetische Strahlung und/oder den Schall und/oder die Wärme und/oder elektromagnetische Felder zu erfassen. Erfindungsgemäß können je nach Fügeaufgabe (Schweißprozess, Schweißstromstärke, Material, etc.) auch nur einzelne Emissionsarten erfasst bzw. verwendet werden. Sensoren können je nach Fügeaufgabe wahlweise ausgewählt und am Helm oder einem anderen Teil der persönlichen Schutzausrüstung montiert werden. Vorteilhafterweise werden die Sensoren automatisch von dem zentralen Prozessor erkannt.

Gemäß einer besonders vorteilhaften Ausgestaltung der vorliegenden Erfindung werden weitere Komponenten eines zur Durchführung des Füge- oder Trennverfahrens eingesetzten Systems und/oder ein oder mehrere externe Geräte über den zentralen Prozessor über Kabel oder kabellos miteinander für einen Datenaustausch geeignet eingerichtet, d.h. vernetzt und ein solcher Datenaustausch durchgeführt.

Beispiel für derartige Komponenten des Systems sind die Stromquelle, die Versorgung mit Zusatzwerkstoff und die Versorgung mit Schutzgas, und alle weiteren nach dem Stand der Technik bekannten Komponenten eines Füge- oder Trennsystems. Ein Schweißsystem ist hier nur ein Beispiel für ein Fügesystem. Als Beispiele für weitere externe Komponenten können ein Bildschirm zur Beobachtung des Füge- oder Trennverfahrens, der z.B. in einem anderen Raum aufgestellt ist, in dem ein Ausbilder oder eine andere Person die das Füge- oder Trennverfahren durchführende Person beobachten kann, oder ein externer Datenspeicher sein, der Daten z.B. für eine Dokumentation speichert.

Die Überprüfung der Qualität der Fügeverbindung erfolgt z.B. direkt durch den zentralen Prozessor, sodass ggf. auch automatisch Maßnahmen über die Ansteuerung der Stromquelle oder anderer Peripherie- und/oder externer Geräte getroffen werden, um eine gleichbleibende Qualität auch bei einer Veränderung der Randbedingungen gewährleisten zu können.

Die persönliche Schutzausrüstung umfasst eine Sende- und Empfangseinheit wie z.B. W-Lan oder Bluetooth oder andere Datenübertragungstechniken. Somit können die Daten der Stromquelle nicht nur an z. B. den im Helm integrierten zentralen Prozessor geschickt werden, sondern es können auch Daten der Helmsensoren genutzt werden, um die Stromquelle oder andere Peripheriegeräte zu kontrollieren und zu steuern. Diese Einheit befindet sich besonders vorteilhaft am Gürtel des Schweißers, um die Strahlenbelastung am Kopf des Schweißers zu minimieren.

Gemäß einer besonders vorteilhaften Weiterbildung der vorliegenden Erfindung wird eine Kamera als Sensor eingesetzt, die so positioniert ist, dass sie den Lichtbogen eines Schweißverfahrens aus nächster Nähe aufnimmt.

Der zentrale Prozessor übernimmt durch die Erfindung vorteilhafterweise die zentrale Rolle im Schweißprozess. Dies wird dadurch möglich, dass die vom Prozess ausgesandten Informationen des Schweißprozesses direkt, d. h. am Lichtbogen, gemessen und dem Schweißer unmittelbar dargestellt werden können sowie dadurch, dass die Daten von dem zentralen Prozessor verarbeitet werden (z.B. Digitalisierung, selektive Darstellung, Kombination, Korrelation etc.). Somit wird eine zeitliche Zuordnung der einzelnen Signale zueinander möglich.

Die persönliche Schutzausrüstung kann auch ein Mikrophon beinhalten, um die Schallemissionen des Schweißprozesses zu erfassen. Dieses Mikrophon lässt sich zudem dafür verwenden, Sprachbefehle des Schweißers zu erfassen. Die Sprachbefehle werden von dem zentralen Prozessor ausgewertet und zur Steuerung des Prozesses genutzt. Dadurch kann sich der Schweißer auf den Prozess konzentrieren und wird nicht abgelenkt.

Die Energieversorgung des zentralen Prozessors erfolgt über eine mitgeführte Batterie, welche sich auch an einem Gürtel befinden kann. Als Alternative oder zusätzlich zur mitgeführten Batterie kann die Energieversorgung auch anhand von Kabeln vom Hausstromnetz erfolgen.

Gemäß einer weiteren besonders vorteilhaften Ausgestaltung der vorliegenden Erfindung werden zwei oder mehr als zwei Sensoren eingesetzt, die zur Erfassung einer Emissionsart geeignet ausgebildet sind.

Besonders vorteilhaft werden zwei oder mehr Sensoren verwendet, um z.B. eine örtliche Auflösung der Emissionsinformationen zu gewährleisten. Hierbei kann auch der Abstand zwischen den zwei oder mehreren Sensoren vergrößert werden, um die dreidimensionale Darstellung der Informationen zu verbessern.

Zwei oder mehr als zwei Sensoren können vorteilhafterweise der Redundanz und damit der Sicherheit dienen, örtlich aufgelöste Daten können erstellt werden, geometrische Daten können so erfasst werden, z.B. durch Triangulation (z.B. 2, 3 oder 4 Sensoren für 2- oder 3-dimensionale Triangulation oder Streifenprojektion). Desweiteren können mit besonderem Vorteil statistische Auswertungen durchgeführt werden, sofern eine dafür erforderliche große Zahl an Sensoren eingesetzt wird.

Gemäß einer bevorzugten Ausgestaltung weist die persönliche Schutzausrüstung, insbesondere ein Schutzhelm oder eine Schutzbrille, ein Display auf, auf dem für die das Füge- oder Trennverfahren durchführende Person Information dargestellt wird, die von dem zentralen Prozessor als Ausgangsdaten zur Verfügung gestellt werden, wobei diese Daten die gesamte vom Sensor aufgenommene Information und/oder einen durch den Algorithmus ausgewählten Teil der Information umfassen.

Besonders vorteilhaft übersteigt der Erfassungsbereich (Frequenz, Auflösung, Dynamik, Messbereich) der Sensoren den der menschlichen Sinnesorgane bzw. den der bisher verwendeten Sensoren.

Die Visualisierung der Emissionsdaten kann durch ein Display im Helm, z.B. LCD, durch Projektion, an einem entfernten Ort oder auch durch eine Brille, welche wahlweise vom Schweißer in Kombination mit einem Helm getragen wird, erfolgen.

Besonders bevorzugt ist das erfindungsgemäße Verfahren dadurch gekennzeichnet, dass die persönliche Schutzausrüstung, insbesondere ein Schutzhelm oder eine Schutzbrille, ein Display aufweist, auf dem für die das Füge- oder Trennverfahren durchführende Person Information dargestellt wird, die von dem zentralen Prozessor als Ausgangsdaten zur Verfügung gestellt werden, wobei auch Eingangsdaten, die von einem Sensor aufgenommene Meßwerte physikalischer Größen darstellen, die vom menschlichen Auge, Gehör oder Geruchssinn nicht wahrnehmbar sind, in für das menschliche Auge oder Gehör wahrnehmbare Ausgangsdaten umgewandelt werden.

Als Beispiele für solche physikalische Größen könne hier Infratrotstrahlung, Laserstrahlung oder geruchlose Gase angegeben werden.

Der zentrale Prozessor ist bevorzugt für eine Datenverarbeitung in Echtzeit geeignet ausgebildet.

Zur Erfassung von gas- und partikelförmigen Emissionen sind Sensoren vorgesehen, die sich vorzugsweise im Atembereich des Schweißers befinden oder die Emissionen im Atembereich des Schweißers bestimmen, sodass die Grenzwerte in-situ bestimmt werden können. Hierbei können diese Sensoren, die Gesamtrauchmenge wie auch die Zusammensetzung z.B. durch Spektroskopie bestimmen.

Die Emissionen können auch gesammelt bzw. aufgefangen und im Labor ausgewertet werden. Insbesondere wichtige Informationen sind CrVI, Cr, Mn, Ni, Co und ähnliche Stoffe. Neu ist hierbei jedoch, dass die im Labor gewonnen Daten mit Hilfe des zentralen Prozessors mit den Informationen der anderen Sensoren korreliert werden, sodass bei zukünftigen Schweißungen anhand der anderen Sensorsignale auf die Emissionsentstehung geschlossen werden kann, ohne diese direkt messen zu müssen. Der zentrale Prozessor kann somit als "selbstlernendes" System verwendet werden. Auch die Grenzwerte der elektromagnetischen Strahlung (insbesondere der UV-Strahlung) werden in-situ überwacht. Besonders vorteilhaft wird der Transmissionsgrad des Sichtfensters im Helm an die Intensität der auftreffenden elektromagnetischen Strahlung angepasst.

Der zentrale Prozessor kann sich auch in einem tragbaren Computer (Tablet) oder in einem Mobiltelefon (Smartphone, Handy) befinden.

In einer weiteren Ausführungsform der Erfindung nimmt der zentrale Prozessor automatisch eine Verbindung mit für den Fügevorgang eingesetzten Geräten und Sensoren, vorzugsweise mit allen für den Fügevorgang notwendigen Geräten und Sensoren, auf und bindet diese, nach Bestätigung durch den Benutzer, in das Kommunikationsnetz mit ein. Hierdurch kommt es zu einer erheblichen Zeitersparnis und Vereinfachung, da alle Geräte von dem zentralen Prozessor überwacht und gesteuert werden. Der Schweißer bzw. allgemein die das Verfahren durchführende Person hat nur ein System, dass er zudem individuell auf seine persönlichen Bedürfnisse anpassen kann. Er hat Zugriff auf wichtige Daten, wie Schweißparameter, WPS, Qualifikation und Handbücher der für die Fügeaufgabe notwendigen Technik und kann somit auch auf alte Einstellungen und Erfahrungen zurückgreifen.

Die Erfindung hat eine Reihe von Vorteilen, von denen hier nur einige wenige nochmal angeführt werden können: Die vorliegende Erfindung hat z.B. den Vorteil, dass die Informationen des Schweißprozesses direkt und unmittelbar am Entstehungsort erfasst werden und die gewonnenen sowie die aufgearbeiteten Informationen unmittelbar durch die Nähe zu den Sinnesorganen des Menschen visualisiert werden können, ohne den Schweißer von seiner Aufgabe abzulenken. Durch die umfassende Erfassung der Emissionen von Schweißprozessen und verwandten Verfahren sowie durch die Datenverarbeitung und die Kombination verschiedener Emissionsarten ergeben sich folgenden Verbesserungen:
- Steigerung der Qualität
- Verbesserte Qualitätskontrolle und dadurch Reduzierung des Kontrollaufwandes und Nacharbeit
- Erhöhung der Informationsdichten durch die Ausweitung der erfassbaren Frequenz, des Erfassungsbereichs und der Auflösung sowie durch die selektive Auswahl und Kombination von wichtigen Informationen und dadurch Vereinfachung des Handlings bzw. der Bedienbarkeit.

## Patentansprüche

1. Verfahren zum Fügen oder Trennen, wobei eine das Füge- oder Trennverfahren durchführende Person mit einer persönlichen Schutzausrüstung ausgestattet ist und wobei zumindest ein Teil das Füge- oder Trennverfahren charakterisierender Daten und/oder zumindest ein Teil der bei der Durchführung des Füge- oder Trennverfahrens entstehenden Emissionen erfasst werden und die erfassten Informationen einem zentralen Prozessor als Eingangsdaten zur Verfügung gestellt werden, und wobei der zentrale Prozessor die Eingangsdaten verarbeitet und Ausgangsdaten erzeugt, **dadurch gekennzeichnet, dass** der zentrale Prozessor in die persönliche Schutzausrüstung integriert ist und die erzeugten Ausgangsdaten zumindest zum Teil dazu eingesetzt werden, um das Verfahren zu steuern, wobei die Steuerung durch die durchführende Person ergänzt wird.

2. Verfahren nach Anspruch 1, **dadurch gekennzeichnet, dass** mindestens ein Sensor zur Erfassung der Emissionen in die persönliche Schutzausrüstung integriert ist.

3. Verfahren nach Anspruch 1 oder 2, **dadurch gekennzeichnet, dass** sich mindestens ein Sensor zur Erfassung der Emissionen an einem Brenner zur Durchführung des Füge- oder Trennverfahrens befindet.

4. Verfahren nach Anspruch 1 bis 3, **dadurch gekennzeichnet, dass** zwei oder mehr Sensoren eingesetzt werden, die jeweils zur meßtechnischen Erfassung einer unterschiedlichen Emissionsart des Füge-oder Trennverfahrens geeignet ausgebildet sind, und die so gewonnenen Messwerte dem zentralen Prozessor als Eingangsdaten zur Verfügung stellt, mit Hilfe eines im Arbeitsspeicher hinterlegten Algorithmus verarbeitet und die durch diese Datenverarbeitung erzeugten Ausgangsdaten zur Steuerung des Verfahrens eingesetzt werden.

5. Verfahren nach einem der vorstehenden Ansprüche, **dadurch gekennzeichnet, dass** weitere Komponenten eines zur Durchführung des Füge- oder Trennverfahrens eingesetzten Systems und/oder ein oder mehrere externe Geräte über den zentralen Prozessor über Kabel oder kabellos miteinander für einen Datenaustausch geeignet eingerichtet, d.h. vernetzt sind, und ein solcher Datenaustausch durchgeführt wird.

6. Verfahren nach einem der vorstehenden Ansprüche, **dadurch gekennzeichnet, dass** eine Kamera als Sensor eingesetzt wird, die so positioniert ist, dass sie den Lichtbogen eines Schweißverfahrens aus nächster Nähe aufnimmt.

7. Verfahren nach einem der vorstehenden Ansprüche, **dadurch gekennzeichnet, dass** zwei oder mehr als zwei Sensoren eingesetzt werden, die zur Erfassung einer Emissionsart geeignet ausgebildet sind.

8. Verfahren nach einem der vorstehenden Ansprüche, **dadurch gekennzeichnet, dass** die persönliche Schutzausrüstung, insbesondere ein Schutzhelm oder eine Schutzbrille, ein Display aufweist, auf dem für die das Füge- oder Trennverfahren durchführende Person Information dargestellt wird, die von dem zentralen Prozessor als Ausgangsdaten zur Verfügung gestellt werden, wobei diese Daten die gesamte vom Sensor aufgenommene Information und/oder einen durch den Algorithmus ausgewählten Teil der Information umfassen.

9. Verfahren nach einem der vorstehenden Ansprüche, **dadurch gekennzeichnet, dass** die persönliche Schutzausrüstung, insbesondere ein Schutzhelm oder eine Schutzbrille, ein Display aufweist, auf dem für die das Füge- oder Trennverfahren durchführende Person Information dargestellt wird, die von dem zentralen Prozessor als Ausgangsdaten zur Verfügung gestellt werden, wobei auch Eingangsdaten, die von einem Sensor aufgenommene Messwerte physikalischer Größen darstellen, die vom menschlichen Auge, Gehör oder Geruchssinn nicht wahrnehmbar sind, in für das menschliche Auge oder Gehör wahrnehmbare Ausgangsdaten umgewandelt werden.

10. Verfahren nach einem der vorhergehenden Ansprüche, **dadurch gekennzeichnet, dass** der zentrale Prozessor automatisch eine Verbindung mit Komponenten eines zur Durchführung des Füge- oder Trennverfahrens eingesetzten Systems, vorzugsweise mit allen Komponenten des zur Durchführung des Füge- oder Trennverfahrens eingesetzten Systems, aufnimmt.

11. Füge- oder Trennsystem zur Durchführung eines Verfahrens zum Fügen oder Trennen nach einem der Ansprüche 1 bis 8, das die folgenden Komponenten aufweist:
- Füge- oder Trennwerkzeug, das von einer das Füge- oder Trennverfahren durchführenden Person geführt wird,
- persönliche Schutzausrüstung, mit der die das Füge- oder Trennverfahren durchführenden Person ausgestattet ist,
- Steuereinrichtung für das Steuern von Prozessparametern,
- Energieversorgung für das Verfahren,
- mindestens einem Sensor zur Erfassung von Emissionen aus dem Verfahren,
- zentraler Prozessor,
- Mittel zum Steuern einzelner Prozessparameter
**dadurch gekennzeichnet, dass**
- der zentrale Prozessor in die persönliche Schutzausrüstung integriert ist und
- die Mittel zum Steuern der Prozessparameter in Wirkverbindung mit dem zentralen Prozessor und in Wirkverbindung mit der das Füge- oder Trennverfahren durchführenden Person stehen.
